# EUROPEAN PATENT SPECIFICATION

(11) **EP 0 320 483 B1**
(45) Date of publication and mention of the grant of the patent: **06.04.1994**
(21) Application number: 88870182.8
(22) Date of filing: 08.12.1988
(51) Int. Cl.: C12N 11/04, A01N 63/00

(54) **Encapsulation method**
Verfahren zur Verkapselung
Procédé d'encapsulation

(30) Priority: 11.12.1987 US 131965
(43) Date of publication of application: 14.06.1989
(73) Proprietor: Monsanto Company, St. Louis Missouri 63167-7020 (US)
(72) Inventor: Baker, Carol Ann, St. Louis Missouri 63146 (US); Brooks, Albert Arthur, St. Louis Missouri 63136 (US); Greenley, Robert Ziolkowski, Frontenac Missouri 63131 (US); Henis, Jay Myls Stuart, Creve Coeur Missouri 63141 (US)
(74) Representative: Ernst, Hubert

(56) References cited:
- EP-A- 0 069 379
- EP-A- 0 202 409
- DE-A- 2 360 647
- US-A- 4 138 290
- G. DURAND et al.: "Les enzymes, production et utilisations industrielles",
- 1983, pages 83-85, Gauthier-Villars; P. MONSAN: "Les méthodesd'immobilisationd'enzymes"

## Description

The present invention relates to encapsulation of biological material. More particularly, the present invention relates to a method of encapsulating biological material such as microbes (bacteria as well as fungi), proteins and peptides in a polymer matrix to enhance the use of the material as agricultural agents (e.g. herbicides, insecticides, etc.) or for some other purpose such as an immobilized catalyst.

The use of microbes as agricultural products has been hampered by the lack of an efficient delivery system for the microbes. A microbe in the fermenter is not "farm-ready". There must be a system for maintaining microbial activity from the fermenter to the farmer and the product must be in a form that is acceptable and useful to the farmer. This may require use in the soil or adhesion to leaf surfaces even through periods of rain.

Five major advantages of the present method for encapsulating microbes or other biological material are: (1) very high recovery of viable active microbes following the encapsulation procedure; (2) the finished product leaves the microbe in a dormant state suitable for storage; (3) the encapsulated microbe formulation is a free-flowing material which can be utilized using standard equipment; (4) encapsulated microbes may be caused to adhere to leaf surfaces; and (5) the present method provides a means to deliver biological material which is subject to photo degradation and/or enzymatically unstable.

Unlike prior art methods such as the alginate bead method of Boshan, ("Alginate Beads as Synthetic Inoculant Carriers for Slow Release of Bacteria that Affect Plant Growth", Applied & Environmental Microbiology Vol 51, No. 5, pp 1089-1098, (1986)), no regrowth step is necessary since usually less than 1.5 logs of cells are lost during the encapsulation process. Since no regrowth step is necessary, the structure of the beads is controlled by the polymer and bead making process and not by the effects of the growing microbe. This results in a more uniform and predictable structure, and release profiles which may be determined by the polymeric composition and microstructure of the beads.

In the alginate bead method of Boshan, viable bacterial numbers dropped 3 to 4 logs when the beads were freeze-dried leaving at most 10⁷ cells/gm of beads. Following the present method, yields of 10⁸ to 10⁹ have been reached without yet reaching the limit of loading capacity. Microbial loadings of 10¹² viable cells per gram of beads should be possible.

### BRIEF DESCRIPTION OF THE DRAWINGS

Figure 1 represents a cross-sectional diagram of a nozzle useful in making beads.

Figure 2 represents a typical PVOH bead in whole and in cross-section.

Figure 3 represents a typical PVP bead.

Figure 4 represents a typical HPC bead.

Figure 5 represents PVOH beads adhering to a leaf surface after rain.

### STATEMENT OF THE INVENTION

The present invention provides a method for encapsulation of biological material in which the biological material is admixed with an aqueous non-ionic polymer solution in which the polymer is present at a concentration of at least 3% w/v. Beads are formed by adding the above mixture dropwise into a water-immiscible non-solvent for the polymer maintained at a temperature sufficient to freeze the bead but not so low to cause freeze fracture of the polymer bead. The polymer beads containing the biological material are then dried to remove substantially all unbound water contained in the beads.

In one aspect the invention enables microbes to become useful agricultural agents. The polymeric beads of the present invention deliver the microbe to the market in a dormant state. Beads can be made which are suitable for delivery to the soil or plant leaves. In addition, the polymeric materials used are biodegradable and the beads can be applied either dry, via a planting or an insecticide box, or wet via a spray nozzle.

Water soluble non-ionic polymers having a solubility of at least 3% w/v and which are not detrimental to the microbe to be encapsulated can be used. By non-ionic polymer is meant a neutral polymer exhibiting substantially no net charge. Polymer concentrations between 5% and 15% are preferred. Suitable polymers include poly(vinyl alcohol) (PVOH), preferably in a M.W. range from about 10,000 to 125,000 and from 85-100% hydrolyzed; polyvinylpyrollidone (PVP), preferably in a M.W. range from about 10,000 to 360,000; dextran, preferably in a M.W. range from about 10,000 to 249,000 and various derivatized cellulose polymers such as hydroxypropylcellulose (HPC), preferably in a M.W. range of about 60,000 to 1,000,000.

Dry PVOH is not readily soluble in cold water, so PVOH beads do not dissolve although swelling and fragmentation can occur. The extent of swelling and fragmentation can be controlled by the molecular weight of the PVOH polymer used. Swelling and fragmentation of PVOH beads are reduced with increased PVOH molecular weight. Dry PVOH is solubilized by heating the aqueous PVOH mixture to a temperature above 80°C, preferably to the boiling point. Beads made with PVOH are substantially insoluble in water at temperatures below 40°C. The integrity of the PVOH beads (rate of a dissolution) can be decreased by blending other cold water soluble polymers, such as polyethylene glycol (PEG) with the PVOH. Beads made from PVP, derivatized cellulose and dextran dissolve when placed in cold or room temperature water.

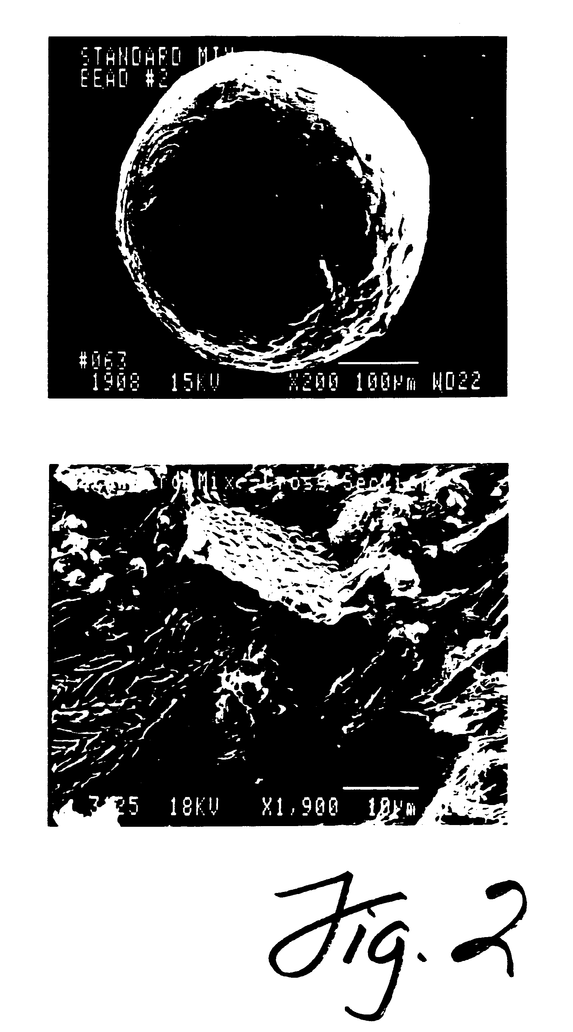

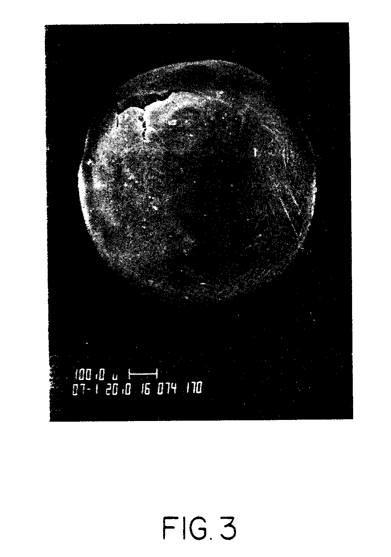

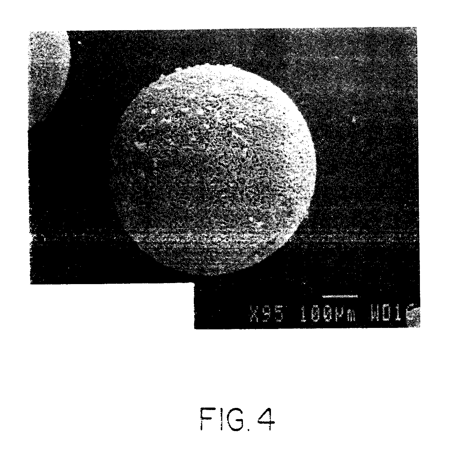

Figure 2 represents a typical PVOH bead in whole and in cross-section showing the sponge-like porous structure. Figure 3 represents a typical (10% w/v) PVP bead. Figure 4 represents a typical (10% w/v, 60,000 m.w.) HPC bead.

The molecular weight of the polymer affects the mechanical strength of the bead. In general, the strength of the bead increases with increasing molecular weight. For example, PVOH beads made from a mixture of various molecular weights but having an average molecular weight of 61,000 swell but fragment very little when added to water. Beads made from 25,000 molecular weight PVOH fragment quickly and dissolve in water. Molecular weights below about 10,000 cause the beads to be very friable. In general, an increase in the molecular weight of the PVOH utilized will decrease the fragmentation. The structural integrity effects can be utilized to alter the release of microbes from the bead matrix. For example, fragmentation of the beads results in quicker release of the biological material contained therein.

One of the unique aspects of the present invention is that the beads can be freeze-dried and retain their morphology. This is possible because the polymers used are water soluble at higher concentrations with lower viscosities than materials currently used for cell immobilization (agar, alginates, etc.). Hence, beads can be made with sufficient polymer (>3% w/v) so that the bead exhibits useful strength upon removal of water. For example, at concentrations of about 5% w/v of PVOH or PVPs viscosities are below 1000 mPa.s (1000 centipoise (cps)). Depending on the polymer and its molecular weight one can increase the polymer concentration to its solubility limit. However, in most cases there will be no useful increase in bead strength above 15% w/v and some of the higher molecular weight polymers are too viscous above this concentration to readily make beads.

Therefore, in another aspect the present invention provides useful compositions comprising a non-ionic water-soluble polymer and biological material, said composition being substantially free of unbound water and having a non-friable sponge-like porous structure. (See Figures 2-4)

In yet another embodiment, the present invention provides a solid composition which comprises a non-ionic polymer, which cannot be readily dissolved at temperatures below 80°C but is dissolvable at temperatures above 80°C, and biological material, said composition substantially free of unbound water and substantially insoluble in water at temperatures below 40°C. In the cases where the biological material is a viable microbe, the microbe content may range between 0.01 and 50 wt% of the composition.

Polymer solutions can be autoclaved at 15 psig, 121°C for 30 minutes. In many cases this is advantageous so that contamination by unwanted microbes can be avoided. Solutions are stirred while cooling to avoid "skinning" on the surface. The "skin" interferes with processing the solution into beads. Nutrients and/or densification agents can be added before autoclaving. Alternately, nutrients can be filter sterilized and added aseptically after autoclaving. Viscosity is preferably maintained in a range of 20 to 1000 mPa.s (20 to 1000 cps). However, the only limitations on the viscosity are dictated by the method of bead making employed.

Since the pH of the polymer solution may change during autoclaving (e.g. acetate residues hydrolyzing from PVOH to form acetic acid in solution), it may be necessary to buffer the polymer solution to maintain a desired pH usually between 6-7.5. Buffers which may be used include any physiologically acceptable buffer which does not adversely affect the microbes. A preferred buffer is 0.05 M sodium phosphate buffer.

Addition of a densification agent is beneficial in most cases to enhance the specific gravity of the formulated bead to make them easier to handle. Any densification agent which does not adversely affect the activity of the microbe may be used. Suitable density agents include silica, silica gel, bentonite and alumina. Alumina is particularly preferred. The alumina is mixed thoroughly with water and a sugar, such as dextrose or sucrose, before adding it to the polymer solution. This permits the alumina to be well dispersed and the sugar pretreats the alumina so that alumina interacts less with any hydroxyl groups on the polymer. This interaction of polymer and alumina can cause the solution to become stringy.

Gram positive and gram negative bacteria as well as fungi can be encapsulated using the method of the present invention. Exemplary microbes include *Bacillus thuringiensis;* microbes of the genus *Pseudomonas* such as *Pseudomonas fluorescens* identified by deposit number NRRL B-15132, NRRL B-15133, NRRL B-15134 and NRRL B-15135, disclosed in US 4,456,684; microbes of the genus *Agrobacterium radiobacter;* and fungal species such as *Alternaria cassiae*. Microbes are added to the polymer solution after it is cooled to room temperature. Cells may be optionally washed of culture supernate although components normally found in culture media and cell products do not interfere with the preparation of the beads. In cases where the microbe may produce a detrimental metabolite, removal by washing is preferred prior to encapsulation. Cells are resuspended in a small amount of fluid prior to mixing with the polymer solution in order to obtain a uniform suspension. The suspending fluid can be simply the supernatant fluid (culture broth) from the initial centrifugation or it can be a specifically defined formulation to enhance the survival of a particular microbe during freezing and freeze drying. In addition, if there are adjuvants that will enhance the microbe's use as a herbicide or pesticide, or enhance its ability to colonize the target plant or soil, these agents may be added. In most cases the volume of suspension fluid is determined such that it is preferably not more than 5% of the polymer solution volume although it may be as high as 20% without difficulty.

Microbes are subject to injury from chilling, freezing and freeze-drying. Unfortunately, it is not fully understood what the injuries are, how these injuries occur, and how to prevent such injuries. Although it has been speculated that gram positive bacteria require less protection than gram negative bacteria, it is perhaps more likely that each microbe must be looked at as a special case. Included in the factors that must be considered are: age of the culture, rate of growth, type of growth medium, magnitude and rate of temperature drop, and the manner of growth following the cold stress. Addition of a sugar, skim milk, glycerol, dimethysulfoxide (DMSO) or other cryoprotective agents can stabilize the cultures. Different cryoprotective agents have different effects and their use is determined empirically. Glycerol, sucrose and DMSO have found wide applicability.

While the above considerations must be made for fungi as well, other treatments must also be made. During the encapsulation process fungal spores are exposed to large amounts of water. Since for a number of fungi the presence of water stimulates germination, it is preferred that the spores be pretreated to protect them from the water during the encapsulation process. It has been found that presoaking the spores in 6M sorbitol is an effective treatment. Spores pretreated with sorbitol in this manner do not wet as easily as untreated spores and survive the encapsulation process. Although pretreatment with sorbitol is preferred, pretreatment of fungal spores with polyethylene glycol (M.W. 200) also works.

Once the above mixture comprising the polymer and microbe or other biological material is complete, the mixture is added drop-wise to a cooling bath at about -30°C. The cooling bath is comprised of a liquid which is water immiscible and a non-solvent for the polymer. It is important that the liquid used in the cooling bath possesses both properties described above. Suitable non-solvents include hexane, petroleum ethers and freon 113. Hexane is particularly preferred as a non-solvent.

The temperature is also an important parameter. If the temperature is too high (e.g. -15°C) beads freeze more slowly and bead morphology is non-uniform. If the temperature is too low (e.g. -80°C) the beads fracture as they form. The non-solvent is preferably stirred during the process to help with heat exchange and to allow large numbers of beads to be made rapidly without sticking together.

The criterion that the non-solvent also be water immiscible substantially prevents the non-solvent from penetrating the bead in concentrations high enough to be toxic to the microbes. In addition, bead formation does not readily occur with water miscible non-solvents since the polymer water mix disperses somewhat into the water miscible non-solvent. This tends to result in the formation of stringy ropes of beads rather than discrete round beads.

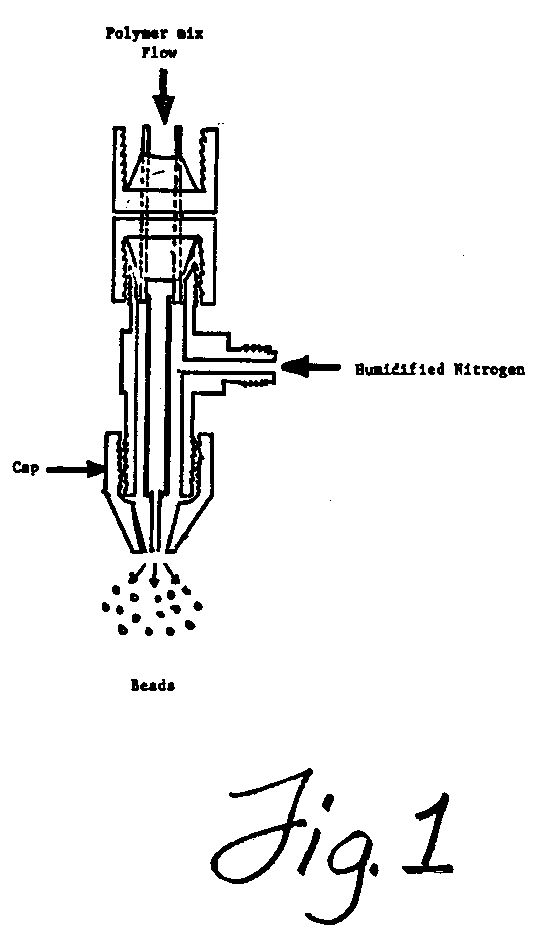

Any method for bead making that does not unduly diminish the viability of the microbe is suitable for use in the present method. Figure 1 represents a nozzle which has been successfully used to make beads with no reduction in cell viability. Variations in the size of openings length of tubing, and the angle of the inside of the cap can be utilized, depending on the viscosities of the polymer/microbial mixture being sprayed and the size of the beads desired. A nozzle is connected to a reservoir of polymer mix which can be held at constant pressure. The pressure used also depends on the viscosity of the polymer mix and the size of beads desired. A sweep gas (preferably humidified nitrogen) enters above the tip and the flow rate is carefully controlled. Control of the sweep gas is vital to controlling bead size. The spray is collected in a chilled non-solvent bath at -30°C. The non-solvent temperature is maintained using a recirculating bath. Since this system involves no high pressure, large pressure drops, heat or ultrasonics to form the beads, it is very gentle to the cells. Substantially no cell damage occurs using this method therefore essentially no loss of viability results.

The frozen beads are collected from the cold non-solvent and dried. Although any means may be used to dry the beads, freeze-drying is preferred for cases in which retention of microbial viability is important. Upon collection from the non-solvent the beads are transferred cold to a freeze dryer. Drying the frozen beads takes from 8-48 hours depending on the configuration of the freeze dryer and the amount of beads to be dried. Incomplete drying results in clumping and loss of bead integrity. Over drying causes loss of viability. The beads are preferably freeze-dried until essentially all unbound water has been removed. In the cases of microbial encapsulation there will be about 1-2 wt% residual water in the dried bead.

The following examples are provided to more fully elucidate the practice of the present invention.

### ADDITION OF DENSITY AGENTS

Beads can be formed with different density agents, including, bentonite, silica, silica gel and alumina. Beads made with 10% w/v PVP plus 10% bentonite increased in density slightly from 0.09 g/ml (10% w/v PVP only) to 0.14 g/ml. Addition of more than 10% bentonite caused the viscosity to increase to a level where the difficulty of handling the more viscous solution outweighed the advantage in increased density of the final product. Silica can be added to at least 20% and at the 20% concentration density of the final product was approximately 0.25 g/ml. At these concentrations the silica did not increase the viscosity to unmanageable levels and was not toxic to the cells being encapsulated. Silica gels were added at the same concentration as the silica with similar results. However, the silica gels used were treated and it was found that the pH of the mixture of polymer, silica gel and cells was much lower. pH changes can adversely affect cells and/or proteins being encapsulated.

Due to its greater density, alumina causes a greater increase in bead density. The preferred type of alumina is alundum which is untreated and does not have a high surface area. Use of high surface area alumina decreased cell viable numbers by more than two logs. Alumina content to 30% may be used in both PVP and PVOH beads. Beads made with the 30% concentration had a density of approximately 1.8 g/ml and were easy to handle with good flow characteristics. Alumina particle sizes of 10 microns or less are preferred to assure good handling characteristics to the polymer mixes to provide good flow through bead making orifices without clogging. In all cases it was necessary to use high speed mixers or blenders to disperse the solids in the polymer solutions or to prewet the solids before addition.

### PRETREATMENT OF ALUMINA

Addition of alumina to poly(vinyl alcohol) solutions resulted in the solution becoming somewhat stringy. It is believed this is due to a coordination effect between the alumina which is capable of electron sharing and the hydroxyl groups on the PVOH. Beads were made by dissolving the polymer in 2/3 of the final amount of water. Five percent dextrose was then added to the remaining water and this solution was used to prewet the alumina. The hydroxyls in the dextrose interacted with the alumina which meant that the alumina interacted less with the polymer when it was combined with the prewet alumina. Sucrose was also used and worked equally well.

### STABILIZATION OF MICROBES

The literature on freeze-drying bacteria has numerous references to the stabilization of bacteria during the freezing and freeze-drying process by means of additives, frequently sugars. In the present process addition of sucrose and dextrose to the polymer cell mixture prior to processing helped to stabilize the gram negative bacteria. Experiments on the encapsulation of *Pseudomonas fluorescens* show that if untreated cells are present at a concentration of 10⁹ per ml, then after encapsulation viability counts were 10⁶ cfu per ml. However, adding 1% glucose or sucrose to the mixture prior to encapsulation resulted in yields as high as 10⁸ cfu/ml from mixtures originally containing 10⁹ cfu/ml.

Encapsulation of the fungus *Alternaria cassiae*, was not successful until a pretreatment for the fungus was devised. Without pretreatment none of the conidia survived the freezing process. With the pretreatment 50% or more of the fungi survived the entire encapsulation procedure. The pretreatment consists of soaking the conidia in a 6 molar sorbitol solution for one to two hours prior to mixing the conidia with the polymer solution. When examined microscopically, spores that were pretreated did not appear to wet as readily as untreated spores. It is believed that the delayed wetting results in less damage to the conidia when freezing occurs. Pretreatment using polyethyleneglycol 200 also works.

### BEAD MAKING

A polymer mix was made consisting of 5% PVOH (78K MW), 1.5% PVOH (125K MW), 3.5% PVOH (10K MW), 0.1% protease peptone #3, 0.05 M Na₂HPO₄ and 20% alumina. The viscosity of this mixture was 360 mPa.s (360 cps). Beads were made using the nozzle illustrated in Figure 1. A reservoir containing the polymer mix was filled and placed under a pressure of 48.3 kPa (7 psig). Pressures of 13.8-103.4 kPa (2-15 psig) have been used depending on the polymer used and size of beads desired. A valve is opened permitting the polymer to flow down to the tip. Sweep gas of humidified nitrogen is used to break up the polymer stream and form the beads. Flow of the sweep gas was 3 standard liters/minute (slpm). Beads were collected in hexane at -30 to -35°C and then freeze-dried. Dried beads were screened and found to have the following distribution: caught on 0.85 mm mesh, 17.2%; on .425 mm mesh, 63.6%; on .250 mm mesh, 16.7% and on .150 mm mesh 2.5%. In another experiment a polymer mixture of 10% PVOH (25K MW), 20% alumina, 0.1% protease peptone #3 with a viscosity of 48.8 mPa.s (48.8 cps) was sprayed with a pressure of 75.85 kPa (11 psig) on the reservoir and 6 slpm of sweep gas. Beads from this run were: 1% on .85mm, 1.8% on .425mm, 32% on .25mm, 40% on 0.125mm, 18% on 0.075mm and 8% less than 0.075mm.

### DOPING PVOH BEADS WITH OTHER POLYMERS

A polymer mix consisting of 5% PVOH (78K MW), 1.5 PVOH (125K MW), 3.5% PVOH (10K MW), 0.1% protease peptone #3, 0.05 M Na₂HPO₄ and 20% alumina was made.

This polymer composition was divided into four 120 gram batches to which was added the following: To batch 1) 2 g of PVP (40K MW); to batch 2) 4 g of PVP (40K MW); to batch 3) 6 gm of PVP (40K MW); and to batch 4) 2 g of PEG8000. Beads were made from these mixtures and compared with beads made with no added polymers in terms of their stability in water. Beads were placed on a glass slide, examined under a stereo microscope at 20X and then a drop of water was added and the dissolution, if any was observed. Batch 1 dissolved more quickly than the undoped beads. Batch 2 and 3 dissolved slightly faster than batch 1 but the difference between 2 and 3 was not detectable. Batch 4 dissolved very quickly.

### ENCAPSULATION FOR SOIL DELIVERY USING A SOYBEAN ROOT COLONIZING BACTERIA

Cells (a strain of *Pseudomonas fluorescens* known to colonize soybean roots) are grown from a standardized inoculum (5 X 10⁻⁷ cfu/ml) for 16 hours. Medium of choice is King's B with 50ug/ml of rifampicin added. Use of a M9 minimal medium results in a drastic decrease in cell viability, as much as five logs following encapsulation. Cells are harvested by centrifugation and resuspended in a small amount of supernatant fluid. In most cases the volume of suspension fluid is determined such that it is preferably not more than 5% of the polymer solution volume although it may be as high as 20% without difficulty. Cells are mixed with a solution containing 10% (w/v) poly(vinyl alcohol) (M.W. 25,000, 98% hydrolyzed, 20% Al₂O₃, 0.1% Protease Peptone #3 (Difco Co.), and 1% dextrose. Immediately after mixing the solution is formed into beads by spraying from a spray nozzle into cold hexane. The beads thus formed are collected from the hexane and freeze dried. Cells viability in the polymer mix was 1.3 X 10¹⁰ cfu/ml. After freeze-drying the beads viability was determined to be 1.3 X 10⁸ cfu/ml. In a typical colonization assay, beads are used to inoculate soil "in furrow" in a pot at rate of 0.2 gram of beads per pot. Controls are cell-free beads (negative control) and liquid inoculum (positive control). Plants (Soybean Williams variety 79) are allowed to grow for two weeks and then the roots are harvested and rhizoplane is assayed for the colonizing bacteria. Results showed that there was no difference in colonization of the roots (as determined by Duncan's Multiple Range Test for Variable), between freshly grown liquid inoculum and encapsulated cells. Both colonized at levels of approximately 2 X 10⁵ cfu/gm of root.

### ENCAPSULATION FOR LEAF DELIVERY USING A LEAF COLONIZING PSEUDOMONAD

Cells of a leaf colonizing pseudomonad were grown and harvested as previously described except two media were used, King's B (without rifampicin) and Nutrient Broth (Difco). Cells grown in the King's B were added to the polymer mix (10% (w/v) PVOH, 25,000 M.W.) at 9.5 X 10⁹ cfu/ml and were present in the finished beads at a concentration of 2 X 10⁹ cfu/ml. Cells grown in nutrient broth were mixed 5.5 X 10⁸ cfu/ml in the polymer mix and were present in the finished beads at a concentration of 2.5 X 10⁷ cfu/ml. These beads are suitable for delivery to the leaf surface in the following manner. The beads are suspended in a PVOH solution ranging in concentration from about .25-10% (w/v) and preferably from about 0.5% to 2% (w/v). The solution may contain other material adapted to enhance the adhesion of the beads to a particular leaf surface. The solution can be sprayed onto the leaf surfaces using conventional spray equipment.

### ENCAPSULATION OF A ABSTRACT SP for SOIL DELIVERY

A *Bacillus sp* having activity against corn root worm was grown in a 1% tryptone-1% glucose medium. Cells were inoculated using 20 ml of a 24 hr. old subculture for every 300 ml of medium. Cells were grown at 30°C for 24 hours at 200 rpm. Cultures were harvested by centrifugation, resuspended in water and mixed with a prepared polymer mix to give final concentrations in the polymer mix of 10% PVP, 30% Al₂O₃ and 2% Protease Peptone #3. Dow Antifoam C (Dow Chemical, Midland, MI) was added to suppress foaming. Beads were made using a spray tip and spraying into hexane at -30°C. Beads were collected from the hexane and freeze-dried. Beads were tested and found to contain 5 X 10⁹ cfu/1.5g of beads. Beads were broadcast onto pots in which corn had been planted. When corn was at an appropriate size corn-root worms were added and corn was allowed to grow. Analysis for damage was done by root weight (the less weight the more damage). Control was beads without cells at a rate of 1.5g/pot. Beads were added at 1.5, 4.5 and 7.5 g/pot. Positive control was Furadan insecticide. Eight replicates were done on each treatment. Results showed the plants exposed to encapsulated cells had significantly higher root weights than the Furadan control when added at the 4.5 and 7.5 gm per pot rate.

### ENCAPSULATION OF BACILLUS THURINGIENSIS var, KURSTAKI

A 200 ml culture of *B. t.* var. *kurstaki* was reduced to a cell paste containing mostly spores and toxin crystals. This paste was resuspended to the original 200 ml volume using sterile water and 1 ml of this suspension was added to 100 ml of a 10% polyvinylalcohol solution. Beads were formed using the above polymer mix as previously described. These beads were tested for activity against tomato hornworm. One gram of beads was suspended in 10 ml of water and 0.05 ml of this solution was painted onto each of six tomato leaves. Leaves were placed in a petri dish with a piece of filter paper moistened with deionized water. Five larvae were added to each dish. A positive control consisted of unencapsulated *B.t.* used at the same concentration and a negative control of beads without spores or toxin crystals. The assay was read after four days. In the assay using beads without cells 27/30 larvae lived. This result indicates that the beads themselves were not toxic to the larvae and did not inhibit their feeding. In the assays with *B. t.* (both encapsulated and unencapsulated) all of the larvae died.

### ENCAPSULATION OF A FUNGUS ALTERNARIA CASSIAE

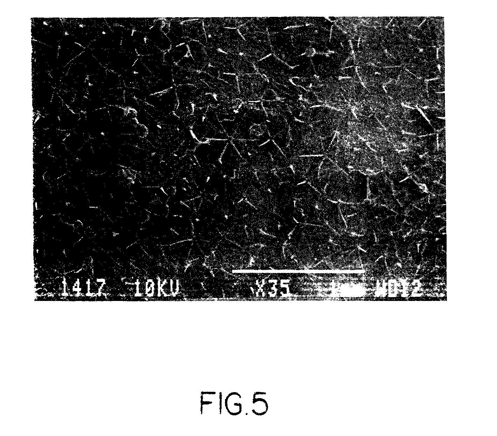

Starting material was conidia of *Alternaria cassiae* in the form of a dry powder. A 0.13 g sample was placed in a test tube with a surfactant in 6 M sorbitol, mixed well to disperse the spores and allowed to stand for approximately one hour. This presoak slows the wetting of the spores when they are placed in the water-soluble polymer solution and enables them to withstand the freezing portion of the encapsulation process. Without this pretreatment all viability is lost. Pretreated spores were mixed with 5% polyvinyl alcohol solution and immediately sprayed into hexane at -30°C to form beads. Beads are freeze-dried until just dry. Overdrying causes loss of viability. Viability of the encapsulated spores varies with the amount of drying and ranges between 20-90%. These beads are mixed with water or desired adjuvants and sprayed onto the surface of sicklepod leaves. Encapsulated spores have a slightly delayed germination and can survive a few hours longer when mixed with water which is an advantage when spraying plants in the field. Poly(vinyl alcohol) beads stick tenaciously to the leaf surface which renders them rain-fast. Encapsulated spores are able to infect and kill sicklepod plants. Referring to Figure 5, PVOH beads are shown adhering to the leaf surface of velvet-leaf even after flushing the leaf surface with water.

## Claims

1. A method for encapsulating biological material which comprises:
a) admixing the biological material with an aqueous non-ionic polymer solution in which the polymer is present at a concentration of at least 3% w/v;
b) forming polymeric beads by adding the mixture of part (a) dropwise into a water-immiscible non-solvent for said polymer, said non-solvent being maintained at a temperature sufficient to freeze the bead but not so low to cause freeze fracture; and
c) drying the beads of step (b) to remove substantially all unbound water in said beads.

2. A method of Claim 1 in which the solution contains polymer at a concentration between 5 and 15% (w/v).

3. A method of Claim 2 in which substantantially all of the unbound water is removed by freeze-drying.

4. A method of Claim 3 which further comprises the presence of a densification agent in the polymer solution.

5. A method of Claim 4 in which the density agent is alumina.

6. A method of Claim 3 in which the polymer is poly(vinyl alcohol).

7. A method of Claim 3 in which the polymer is polyvinylpyrollidone.

8. A method of Claim 3 in which the polymer is dextran.

9. A method of Claim 3 in which the polymer is derivatized cellulose.

10. A method of Claim 3 in which the biological material is a microbe.

11. A method of Claim 10 in which the microbe is a bacteria.

12. A method of Claim 10 in which the microbe is a fungus and the fungus is pretreated to delay wetting during the encapsulation.

13. A method of Claim 11 in which the bacteria is a strain of *Bacillus thuringiensis*.

14. A method of Claim 11 in which the bacteria is a *Pseudomonas sp*.

15. A method of Claim 11 in which the fungus is *Alternaria cassiae*.

16. A method of Claim 3 in which the biological material is a protein.

17. A method of Claim 3 in which the biological material is a peptide.

18. A method of Claim 14 in which the bacteria is selected from the group consisting of *Pseudomonas fluorescens* NRRL B-15132, NRRL B-15133, NRRL B-15134 and NRRL B-15135.

19. A method of Claim 11 in which the bacteria is *Agrobacterium radiobacter*.

20. A method of Claim 14 in which the bacteria is *Pseudomonas fluorescens*.

21. A composition comprising a water-soluble non-ionic polymer and biological material, said composition being substantially free of unbound water and having a non-friable, sponge-like, porous structure.

22. A composition of Claim 21 in which the biological material is a viable microbe.

23. A composition of Claim 21 in which the biological material is selected from the group consisting of proteins and peptides.

24. A composition of Claim 21 further comprising a densification agent.

25. A composition of Claim 21 in which the polymer is selected from the group consisting of poly(vinyl alcohol), polyvinylpyrollidone, dextran and derivatized cellulose.

26. A solid composition comprising a non-ionic polymer which cannot be readily dissolved at temperatures below 80°C but is dissolvable at temperatures above 80°C and a viable microbe, said composition substantially free of unbound water and substantially insoluble in water at temperatures below 40°C.

27. A composition of Claim 26 in which the polymer is poly(vinyl alcohol).

28. A composition of Claim 26 having a microbe content between about 0.01 and 50 wt% of said composition.

29. A composition of Claim 26 further comprising a densification agent.

30. A composition of Claim 29 in which the densification agent is alumina.

31. A method of Claim 5 in which the alumina is relatively low surface area and untreated.

32. A method of Claim 5 in which the alumina has an average particle size of less than 10 microns.

33. A method of Claim 5 in which the alumina is pretreated with a sugar or other agent to reduce the interaction of the alumina with hydroxyl groups on the polymer.

34. A composition of Claim 30 in which the alumina is relatively low surface area and untreated.

35. A composition of Claim 30 in which the alumina has an average particle size of less than 10 microns.

36. A composition of Claim 30 in which the alumina is pretreated with a sugar or other agent to reduce the interaction of the alumina with hydroxyl groups on the polymer.

37. A method of applying biological material to the leaf surface of plants which comprises spraying the leaf surfaces with a mixture comprising the composition of Claim 26 suspended in an aqeous solution of poly(vinyl alcohol).

38. A method of Claim 37 in which the poly (vinyl alcohol) solution has a concentration between about 0.25 and 10% (w/v).

39. A method of Claim 37 in which the poly (vinyl alcohol) solution has a concentration between about 0.5 and 2% (w/v).

40. A method of applying biological material to the leaf surface of plants which comprises spraying the leaf surfaces with a mixture comprising the composition of Claim 27 suspended in an aqueous solution of poly(vinyl alcohol).

41. A method of Claim 40 in which the poly (vinyl alcohol) solution has a concentration between about 0.25 and 10% (w/v).

42. A method of Claim 40 in which the poly (vinyl alcohol) solution has a concentration between about 0.5 and 2% (w/v).

43. A composition which comprises the composition of Claim 26 suspended in an aqueous poly(vinyl alcohol) solution.

44. A composition of Claim 43 in which the poly(vinyl alcohol) solution has a concentration of 0.25 to 10% (w/v).

45. A composition of Claim 43 in which the poly(vinyl alcohol) solution has a concentration of 0.5 to 2.0% (w/v).

## Patentansprüche

1. Verfahren zur Einkapselung von biologischem Material, welches umfaßt:
a) Mischen des biologischen Materials mit einer wässerigen nicht-ionischen Polymer-Lösung, in der das Polymer in einer Konzentration von zumindest 3 % M/V vorliegt;
b) Bilden von Polymerkügelchen durch das tropfenweise Zusetzen der Mischung von Teil (a) in ein mit Wasser nicht mischbares Nicht-Lösungsmittel für das Polymer, wobei das Nicht-Lösungsmittel bei einer Temperatur gehalten wird, die ausreicht, um das Kügelchen zu gefrieren, jedoch nicht so niedrig ist, um einen Gefrierbruch zu bewirken; und
c) Trocknen der Kügelchen von Schritt (b) zur Entfernung im wesentlichen des gesamten ungebundenen Wassers in den Kügelchen.

2. Verfahren nach Anspruch 1, bei welchem die Lösung Polymer in einer Konzentration zwischen 5 und 15 % (M/V) enthält.

3. Verfahren nach Anspruch 2, bei welchem im wesentlichen das gesamte ungebundene Wasser durch Gefriertrocknen entfernt wird.

4. Verfahren nach Anspruch 3, welches weiters das Vorliegen eines Verdichtungsmittels in der Polymer-Lösung umfaßt.

5. Verfahren nach Anspruch 4, bei welchem das Verdichtungsmittel Aluminiumoxid ist.

6. Verfahren nach Anspruch 3, bei welchem das Polymer Poly(vinylalkohol) ist.

7. Verfahren nach Anspruch 3, bei welchem das Polymer Polyvinylpyrrolidon ist.

8. Verfahren nach Anspruch 3, bei welchem das Polymer Dextran ist.

9. Verfahren nach Anspruch 3, bei welchem das Polymer derivatisierte Cellulose ist.

10. Verfahren nach Anspruch 3, bei welchem das biologische Material eine Mikrobe ist.

11. Verfahren nach Anspruch 10, bei welchem die Mikrobe ein Bakterium ist.

12. Verfahren nach Anspruch 10, bei welchem die Mikrobe ein Fungus ist und der Fungus vorbehandelt wird, um ein Netzen während der Einkapselung zu verzögern.

13. Verfahren nach Anspruch 11, bei welchem das Bakterium ein Stamm von Bacillus thuringiensis ist.

14. Verfahren nach Anspruch 11, bei welchem das Bakterium ein Pseudomonas sp. ist.

15. Verfahren nach Anspruch 11, bei welchem der Fungus Alternaria cassiae ist.

16. Verfahren nach Anspruch 3, bei welchem das biologische Material ein Protein ist.

17. Verfahren nach Anspruch 3, bei welchem das biologische Material ein Peptid ist.

18. Verfahren nach Anspruch 14, bei welchem das Bakterium aus der Gruppe bestehend aus Pseudomonas fluorescens NRRL B-15132, NRRL B-15133, NRRL B-15134 und NRRL B-15135 ausgewählt wird.

19. Verfahren nach Anspruch 11, bei welchem das Bakterium Agrobacterium radiobacter ist.

20. Verfahren nach Anspruch 14, bei welchem das Bakterium Pseudomonas fluorescens ist.

21. Zusammensetzung, welche ein wasserlösliches nicht-ionisches Polymer und biologisches Material umfaßt, welche Zusammensetzung im wesentlichen frei von ungebundenem Wasser ist und eine nichtbrüchige, schwammige, poröse Struktur aufweist.

22. Zusammensetzung nach Anspruch 21, worin das biologische Material eine lebensfähige Mikrobe ist.

23. Zusammensetzung nach Anspruch 21, worin das biologische Material aus der Gruppe bestehend aus Proteinen und Peptiden ausgewählt ist.

24. Zusammensetzung nach Anspruch 21, welche weiters ein Verdichtungsmittel umfaßt.

25. Zusammensetzung nach Anspruch 21, worin das Polymer aus der Gruppe bestehend aus Poly-(vinylalkohol), Polyvinylpyrrolidon, Dextran und derivatisierter Cellulose ausgewählt ist.

26. Feste Zusammensetzung, welche ein nicht-ionisches Polymer, das bei Temperaturen von weniger als 80°C nicht leicht gelöst werden kann, jedoch bei Temperaturen von mehr als 80°C löslich ist, und eine lebensfähige Mikrobe umfaßt, welche Zusammensetzung im wesentlichen frei von ungebundenem Wasser und in Wasser bei Temperaturen von weniger als 40°C im wesentlichen unlöslich ist.

27. Zusammensetzung nach Anspruch 26, worin das Polymer Poly-(vinylalkohol) ist.

28. Zusammensetzung nach Anspruch 26, mit einem Mikrobengehalt zwischen etwa 0,01 und 50 % Masse der Zusammensetzung.

29. Zusammensetzung nach Anspruch 26, welche weiters ein Verdichtungsmittel umfaßt.

30. Zusammensetzung nach Anspruch 29, worin das Verdichtungsmittel Aluminiumoxid ist.

31. Verfahren nach Anspruch 5, bei welchem das Aluminiumoxid eine relativ kleine Oberfläche aufweist und unbehandelt ist.

32. Verfahren nach Anspruch 5, bei welchem das Aluminiumoxid eine mittlere Teilchengröße von weniger als 10 µm aufweist.

33. Verfahren nach Anspruch 5, bei welchem das Aluminiumoxid mit einem Zucker oder anderen Mittel vorbehandelt wird, um die Wechselwirkung des Aluminiumoxids mit Hydroxylgruppen am Polymer zu reduzieren.

34. Zusammensetzung nach Anspruch 30, bei welchem das Aluminiumoxid eine relativ kleine Oberfläche aufweist und unbehandelt ist.

35. Zusammensetzung nach Anspruch 30, bei welchem das Aluminiumoxid eine mittlere Teilchengröße von weniger als 10 µm aufweist.

36. Zusammensetzung nach Anspruch 30, bei welchem das Aluminiumoxid mit einem Zucker oder anderen Mittel vorbehandelt wird, um die Wechselwirkung des Aluminiumoxids mit Hydroxylgruppen am Polymer zu reduzieren.

37. Verfahren zum Aufbringen von biologischem Material auf die Blattfläche von Pflanzen, welches das Besprühen der Blattflächen mit einer Mischung, die die Zusammensetzung nach Anspruch 26 suspendiert in einer wässerigen Lösung von Poly-(vinylalkohol) enthält, umfaßt.

38. Verfahren nach Anspruch 37, bei welchem die Poly-(vinylalkohol)-Lösung eine Konzentration zwischen etwa 0,25 und 10 % (M/V) aufweist.

39. Verfahren nach Anspruch 37, bei welchem die Poly-(vinylalkohol)-Lösung eine Konzentration zwischen etwa 0,5 und 2 % (M/V) aufweist.

40. Verfahren zum Aufbringen von biologischem Material auf die Blattfläche von Pflanzen, welches das Besprühen der Blattflächen mit einer Mischung, die die Zusammensetzung nach Anspruch 27 suspendiert in einer wässerigen Lösung von Poly-(vinylalkohol) enthält, umfaßt.

41. Verfahren nach Anspruch 40, bei welchem die Poly-(vinylalkohol)-Lösung eine Konzentration zwischen etwa 0,25 und 10 % (M/V) aufweist.

42. Verfahren nach Anspruch 40, bei welchem die Poly-(vinylalkohol)-Lösung eine Konzentration zwischen etwa 0,5 und 2 % (M/V) aufweist.

43. Zusammensetzung, welche die Zusammensetzung nach Anspruch 26 suspendiert in einer wässerigen Poly-(vinylalkohol)-Lösung umfaßt.

44. Zusammensetzung nach Anspruch 43, worin die Poly-(vinylalkohol)-Lösung eine Konzentration von 0,25 bis 10 % (M/V) aufweist.

45. Zusammensetzung nach Anspruch 43, worin die Poly-(vinylalkohol)-Lösung eine Konzentration von 0,5 bis 2 % (M/V) aufweist.

## Revendications

1. Procédé d'encapsulation d'une substance biologique, qui comporte :
a) le fait de mélanger la substance biologique avec une solution aqueuse d'un polymère non-ionique, dans laquelle la concentration du polymère vaut au moins 3 % p/v ;
b) le fait de former des perles de polymère, en ajoutant goutte à goutte le mélange provenant de l'étape (a) dans un non-solvant dudit polymère, non-miscible à l'eau, ledit non-solvant étant maintenu à une température suffisamment basse pour que les perles soient congelées, mais non pas assez basse pour provoquer la rupture des perles lors de leur congélation ; et
c) le fait de faire sécher les perles provenant de l'étape (b), pour chasser pratiquement toute l'eau non-liée présente dans lesdites perles.

2. Procédé conforme à la revendication 1, dans lequel la concentration du polymère dans la solution vaut entre 5 et 15 % (p/v).

3. Procédé conforme à la revendication 2, dans lequel pratiquement toute l'eau non-liée est chassée par lyophilisation.

4. Procédé conforme à la revendication 3, comportant en outre la présence d'un agent de densification dans la solution de polymère.

5. Procédé conforme à la revendication 4, dans lequel l'agent de densification est de l'alumine.

6. Procédé conforme à la revendication 3, dans lequel le polymère est du poly(alcool vinylique).

7. Procédé conforme à la revendication 3, dans lequel le polymère est de la poly(vinylpyrrolidone).

8. Procédé conforme à la revendication 3, dans lequel le polymère est du dextrane.

9. Procédé conforme à la revendication 3, dans lequel le polymère est un dérivé de cellulose.

10. Procédé conforme à la revendication 3, dans lequel la substance biologique est un microbe.

11. Procédé conforme à la revendication 10, dans lequel le microbe est une bactérie.

12. Procédé conforme à la revendication 10, dans lequel le microbe est un champignon inférieur, et l'on fait subir à ce champignon inférieur un traitement préalable pour retarder le mouillage au cours de l'encapsulation.

13. Procédé conforme à la revendication 11, dans lequel la bactérie est une souche de *Bacillus thuringiensis*.

14. Procédé conforme à la revendication 11, dans lequel la bactérie est une *Pseudomonas sp*.

15. Procédé conforme à la revendication 11, dans lequel le champignon inférieur est *Alternaria cassiae*.

16. Procédé conforme à la revendication 3, dans lequel la substance biologique est une protéine.

17. Procédé conforme à la revendication 3, dans lequel la substance biologique est un peptide.

18. Procédé conforme à la revendication 14, dans lequel la bactérie est choisie dans le groupe constitué par *Pseudomonas fluorescens* NRRL B-15132, NRRL B-15133, NRRL B-15134 et NRRL B-15135.

19. Procédé conforme à la revendication 11, dans lequel la bactérie est *Agrobacterium radiobacter*.

20. Procédé conforme à la revendication 14, dans lequel la bactérie est *Pseudomonas fluorescens*.

21. Composition comprenant un polymère hydrosoluble non-ionique et une substance biologique, ladite composition ne contenant pratiquement pas d'eau non-liée et présentant une structure non-friable et poreuse, semblable à celle d'une éponge.

22. Composition conforme à la revendication 21, dans laquelle la substance biologique est un microbe viable.

23. Composition conforme à la revendication 21, dans laquelle la substance biologique est choisie dans le groupe constitué par les protéines et les peptides.

24. Composition conforme à la revendication 21, comprenant en outre un agent de densification.

25. Composition conforme à la revendication 21, dans laquelle le polymère est choisi dans le groupe constitué par le poly(alcool vinylique), la poly(vinylpyrrolidone), le dextrane et les dérivés de la cellulose.

26. Composition solide comprenant un polymère non-ionique, que l'on ne peut pas facilement dissoudre à des températures inférieures à 80°C, mais que l'on peut dissoudre à des températures supérieures à 80°C, et un microbe viable, ladite composition étant pratiquement exempte d'eau non-liée et pratiquement insoluble dans l'eau à des températures inférieures à 40°C.

27. Composition conforme à la revendication 26, dans laquelle le polymère est du poly(alcool vinylique).

28. Composition conforme à la revendication 26, dont la teneur en microbe vaut entre environ 0,01 et 50 % en poids de ladite composition.

29. Composition conforme à la revendication 26, comprenant en outre un agent de densification.

30. Composition conforme à la revendication 29, dans laquelle l'agent de densification est de l'alumine.

31. Procédé conforme à la revendication 5, dans lequel l'alumine présente une aire spécifique relativement faible et n'est pas traitée.

32. Procédé conforme à la revendication 5, dans lequel la taille moyenne des particules d'alumine vaut moins de 10 µm.

33. Procédé conforme à la revendication 5, dans lequel on a fait subir un traitement préalable à l'alumine, avec un sucre ou un autre agent, pour affaiblir l'interaction de l'alumine avec les groupes hydroxyle du polymère.

34. Composition conforme à la revendication 30, dans laquelle l'alumine présente une aire spécifique relativement faible et n'est pas traitée.

35. Composition conforme à la revendication 30, dans laquelle la taille moyenne des particules d'alumine vaut moins de 10 µm.

36. Composition conforme à la revendication 30, dans laquelle on a fait subir un traitement préalable à l'alumine, avec un sucre ou un autre agent, pour affaiblir l'interaction de l'alumine avec les groupes hydroxyle du polymère.

37. Procédé d'application d'une substance biologique sur la surface de feuilles de plantes, qui comporte le fait de pulvériser, sur la surface des feuilles, un mélange comprenant une composition conforme à la revendication 26, en suspension dans une solution aqueuse de poly(alcool vinylique).

38. Procédé conforme à la revendication 37, dans lequel la concentration du poly(alcool vinylique) dans la solution vaut entre environ 0,25 et 10 % (p/v).

39. Procédé conforme à la revendication 37, dans lequel la concentration du poly(alcool vinylique) dans la solution vaut entre environ 0,5 et 2 % (p/v).

40. Procédé d'application d'une substance biologique sur la surface de feuilles de plantes, qui comporte le fait de pulvériser, sur la surface des feuilles, un mélange comprenant une composition conforme à la revendication 27, en suspension dans une solution aqueuse de poly(alcool vinylique).

41. Procédé conforme à la revendication 40, dans lequel la concentration du poly(alcool vinylique) dans la solution vaut entre environ 0,25 et 10 % (p/v).

42. Procédé conforme à la revendication 40, dans lequel la concentration du poly(alcool vinylique) dans la solution vaut entre environ 0,5 et 2 % (p/v).

43. Composition qui comporte une composition conforme à la revendication 26, en suspension dans une solution aqueuse de poly(alcool vinylique).

44. Composition conforme à la revendication 43, dans laquelle la concentration du poly(alcool vinylique) dans la solution vaut entre environ 0,25 et 10 % (p/v).

45. Composition conforme à la revendication 43, dans laquelle la concentration du poly(alcool vinylique) dans la solution vaut entre environ 0,5 et 2 % (p/v).
